(19) Europäisches Patentamt
European Patent Office
Office européen des brevets

(11) Veröffentlichungsnummer: **0 226 866 B1**

(12) **EUROPÄISCHE PATENTSCHRIFT**

(45) Veröffentlichungstag der Patentschrift: **11.09.91**

(51) Int. Cl.⁵: **C07D 225/08**, //A61K31/395

(21) Anmeldenummer: **86116516.5**

(22) Anmeldetag: **27.11.86**

# (54) Verfahren zur Isomerisierung von Asocainol.

(30) Priorität: **28.11.85 DE 3541994**

(43) Veröffentlichungstag der Anmeldung:
**01.07.87 Patentblatt 87/27**

(45) Bekanntmachung des Hinweises auf die Patenterteilung:
**11.09.91 Patentblatt 91/37**

(84) Benannte Vertragsstaaten:
**AT CH DE FR GB IT LI**

(56) Entgegenhaltungen:
**EP-A- 0 035 360**
**DE-A- 3 419 099**

**CHEMICAL ABSTRACTS, Band 101, Nr. 17, 22. Oktober 1984, Columbus, Ohio, USA; H. Langenfeld et al.: "Electrophysological profile of the antirhythmic compound asocainolstudied on perfused guinea pig hearts and on isolated cardiac preparations" Seite 44, Spalte 2, Zusammenfassung-Nr. 143 792z**

(73) Patentinhaber: **GÖDECKE AKTIENGESELLSCHAFT**
**Salzufer 16**
**W-1000 Berlin 10(DE)**

(72) Erfinder: **Geibel, Wolfram, Dr.**
**Schönbergweg 2**
**W-7830 Emmendingen 13(DE)**
Erfinder: **Herrmann, Wolfgang, Dr.**
**Zum Baumgarten 13**
**W-7802 Merzhausen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 226 866 B1

## Beschreibung

Gegenstand der vorliegenden Erfindung ist ein neues Verfahren zur Isomerisierung von Asocainol-Isomeren.

(+)-Asocainol oder [(+)-2,12-Dimethoxy-1-hydroxy-7-methyl-6-phenethyl-5,6, 8,9-tetrahydro-7H-dibenz-(d,f)azonin] oder (R,R-Asocainol) ist ein aus der DE-PS 30 07 710 bekannter Arzneimittelwirkstoff, der sich hervorragend zur Blockade unerwünschter Reizbildung und Reizleitung im Nervengewebe eignet. Er ist deshalb besonders zur Localanaesthesie und zur Therapie von Herzrhythmusstörungen geeignet.

Bei der Synthnese von (+)-Asocainol gemäß Beispiel 10 der DE-PS 30 07 710 aus natürlichem Thebain (Merck Index 1976 No. 8988) erhält man das Hydrochlorid in einer Ausbeute von 21 % der Theorie. Neben dem gewünschten (+)-Asocainol mit der Konfiguration (R,R) entsteht in dem äußerst ungünstigen Gewichtsverhältnis von 1:3,25 das wirkungslose und daher unbrauchbare Diastereomere der Konfiguration (R,S). Alle Versuche, dieses ungünstige Isomerenverhältnis durch Veränderung der Synthese-Parameter, wie z. B. durch Einsatz anderer Lösungsmittel und Variation der Temperatur, der Mengenverhältnisse oder der Konzentration zugunsten einer Ausbeutesteigerung des gewünschten Isomeren zu verschieben, blieben erfolglos.

In der DE-P 34 19 099.7 ist beschrieben, daß auch das durch thermische Isomerisierung aus dem unwirksamen R,S-Isomeren herstellbare (-)-Asocainol (das S,S-Isomere) ebenfalls eine antiarrhythmische Wirksamkeit aufweist. Bei der synthetischen Bildung des Racemates (R,R; S,S) aus gleichen Teilen Asocainol in der (R,R)- und (S,S)-Form ergab sich dann ein überraschender pharmakologischer Befund: Anstelle der erwarteten additiven Wirkung wurde ein starker synergistischer antiarrhythmischer Effekt gefunden, wodurch eine erhebliche Wirksamkeitsverbesserung gegenüber (+)-Asocainol erreicht worden ist.

Bei der in der DE-P 34 19 099.7 beschriebenen thermischen Umlagerung bei Temperaturen von 130-200 °C in der Schmelze oder in hochsiedenden Lösungsmitteln entsteht im Gleichgewicht mit dem Ausgangsprodukt in ca. 40 % Ausbeute das S,S-Enantiomere, das durch fraktionierte Kristallisation, z. B. als Hydrochlorid, abgetrennt werden kann. Durch Rückführung des nicht umgesetzten R,S-Isomeren kann praktisch alles R,S-Isomere in die S,S-Form überführt werden und somit - bezogen auf das ursprünglich eingesetzte Ausgangsprodukt Thebain - neben ca. 17-20 % (+)-Asocainol (R,R) noch ca. 45-50 % (-)-Asocainol (S,S) gewonnen werden.

Da jedoch, wie oben gesagt, die höchste Wirkung nicht den Isomeren, sondern dem Racemat zukommt,
bedeutet dies einen unerwünschten Überschuß an S,S-Isomeren.

Es stellte sich daher die Aufgabe, ein Verfahren zur Isomerisierung von Asocainol zu finden, bei dem auch das (+)-Asocainol (R,R) gewonnen werden kann, um somit mit möglichst hoher Ausbeute das racemische (±)-Asocainol herzustellen.

Die Aufgabe wurde durch katalytische Isomerisierung des R,S-Isomeren zum (+)-Asocainol (R,R) gelöst. Dadurch kann die Ausbeute an dem zur Herstellung des racemischen (±)-Asocainols notwendigen (+)-Asocainol von bisher 20 % auf einen beliebigen bzw. erforderlichen Wert erhöht werden.

(+)-Asocainol besitzt gemäß Röntgenstrukturanalyse eine absolute Konfiguration gemäß Formel I.

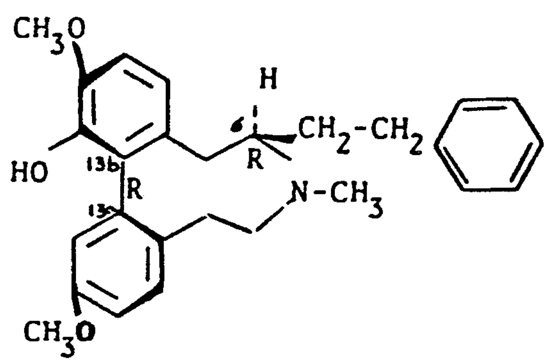

(I)

Theoretisch kann Asocainol, wie schon in DE-P 34 19 099.7 dargelegt, in vier verschiedenen Formen auftreten, die durch

1. das asymmetrische C-Atom in Position 6 und
2. eine Biphenyl-Asymmetrie an der Bindung C 13a-C 13 b

bedingt sind. Die Biphenyl-Asymmetrie wird dadurch hervorgerufen, daß die beiden Phenylringe nicht in einer Ebene liegen, sondern nahezu senkrecht zueinander angeordnet sind. Demnach sind folgende Konfigurationen denkbar:

(R,R) (S,S) (R,S) (S,R)

Das Paar (R,R) und (S,S) sowie das Paar (R,S) und (S,R) sind Enantiomere, die theoretisch zusammen je ein Racemat bilden. Da sich Enantiomere in ihrer Struktur wie Bild und Spiegelbild verhalten, sind sie in allen physikalischen Eigenschaften außer im Vorzeichen der Drehung des polarisierten Lichtes gleich.

Wegen des Vorliegens zweier chiraler Zentren tritt bei Asocainol neben der optischen "Isomerie" auch die sogenannte Diastereomerie auf. Diastereomere sind dadurch gekennzeichnet, daß sie keine Bild-Spiegelbild-Beziehung zueinander aufweisen.

So ist das Paar (R,R) und (S,S) diastereomer zu dem Paar (S,R) und (R,S). Ebenso ist das Racemat (R,R-S,S) ein Diastereomeres des Racemates (S,R - R,S). Die bei den Paaren erstgenannte Konfiguration bezieht sich jeweils auf die Biphenyl-Asymmetrie, die zweite auf das asymmetrische C-Atom in Position 6. (S,R) bedeutet: S-Konfiguration bezüglich der Biphenyl-Asymmetrie, R-Konfiguration am C6.

Im folgenden Formelschema II sind die vier möglichen Konfigurationen und deren Racemate symbolisch wiedergegeben. Zusätzlich ist jeweils die optische Drehung angegeben.

II.

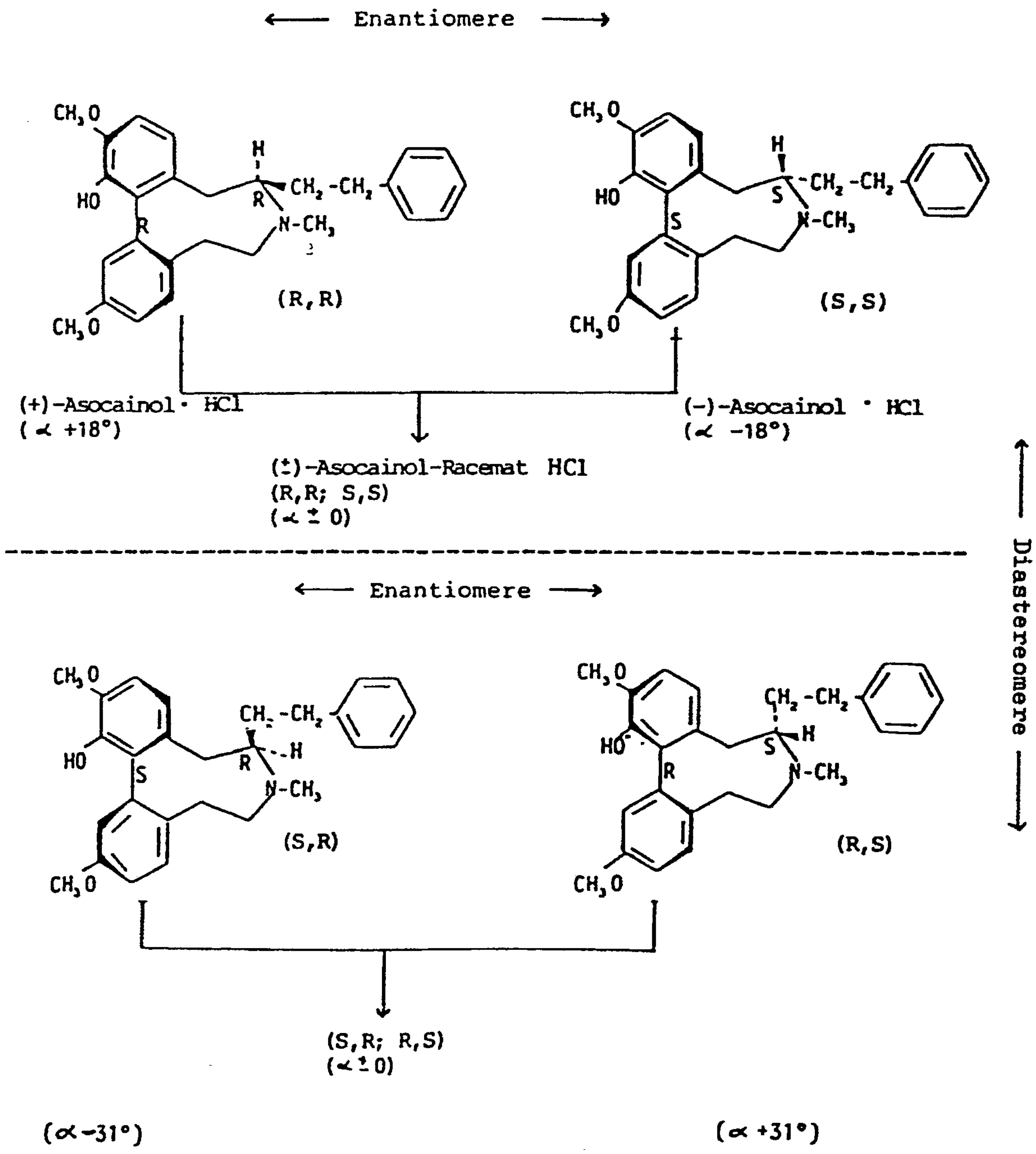


Ausgehend von dem in Überschuß gegenüber dem R,R-Isomeren anfallenden R,S-Isomeren bzw. dem daraus erhältlichen S,S-Isomeren waren verschiedene Wege einer Isomerisierung zur Gewinnung von zusätzlichem R,R-Isomeren bzw. des gewünschten Racemats theoretisch denkbar:

1. Racemisierung der R,S-Verbindung zum diastereomeren (S,R; R,S)-Racemat und anschließende thermische Umlagerungen dieses Racemats in racemisches Asocainol gemäß DE-P 34 19 099.7.
2. Inversion der R,S-Verbindung zur S,R-Verbindung und thermische Umlagerung dieser Verbindung zum (+)-Asocainol gemäß DE-P 34 19 099.7.
3. Änderung der Konfiguration am optisch aktiven C6-Atom der R,S-Verbindung von der S- zur R-Konfiguration, wodurch (+)-Asocainol gebildet wird.
4. Gleichzeitige Änderung der Konfiguration am optisch aktiven C6-Atom der R,S-Verbindung von der S-zur R-Konfiguration, wodurch (+)-Asocainol gebildet wird, und Umlagerung des Biphenyl-Systems der R,S-Verbindung von der R- in die S-Konfiguration zu (-)-Asocainol unter direkter Bildung des racemischen Asocainols. Beide Reaktionen müßten mit gleicher Geschwindigkeit ablaufen.

Essentieller Schritt bei allen vier Wegen ist die Änderung der Konfiguration des optisch aktiven C6-Atoms, welches außer C und H als vierten Substituenten ein tertiäres Amin trägt.

Die Wege 1. und 2. waren nicht gangbar, da R,S-Asocainol weder unter Bildung von S,R-Asocainol racemisiert noch isomerisiert werden konnte.

Wir fanden aber überraschenderweise, daß sich die R,S-Verbindung in Form ihres Hydrochlorids in wäßriger Lösung beim Erhitzen im Druckgefäß auf über 100 °C teilweise in R,R- und S,S-Asocainol umlagert, entsprechend dem in Punkt 4 skizzierten Weg. Die Gesamtausbeute beträgt 50-60 %. Auch das Hydrojodid ist mit gleichem Ergebnis zur Umwandlung gemäß Weg 4. geeignet. Die praktische Anwendung der Umlagerung von R,S-Asocainol zu racemischen Asocainol nach Weg 4. war jedoch nicht gegeben, da die Isomeren immer in einem von 1 abweichenden Verhältnis anfallen. Unsere Versuche, durch Veränderung der Reaktionsbedingungen gleiche Mengen an (+)- und (-)-Asocainol zu erhalten, hatten keinen Erfolg. Wegen der umgleichen Mengenverhältnisse gestaltete sich die Isolierung des racemischen Asocainols, d. h. seine optische Reinigung, äußerst schwierig und aufwendig und war mit größeren Verlusten an Ausbeute verbunden.

Die Lösung der technischen Aufgabe wurde schließlich nach Weg 3. gefunden, sie wird im folgenden näher erläutert.

Nach den vorliegenden Erfahrungen mit dem Hydrochlorid der R,S-Form war zu erwarten, daß bei Verwendung organischer Säuren eine ähnliche Umlagerung eintritt. Wir fanden jedoch sowohl mit Trifluoressigsäure, als auch mit Essigsäure eine Inversion nur am optisch aktiven C6-Atom, d. h. eine Umwandlung der R,S- in die R,R-Form, ohne daß dabei die Konfiguration am Biphenylsystem beeinflußt wurde.

Diese Umwandlung ist bereits bei Temperaturen um 100 °C zu beobachten. Als Lösungsmittel sind Wasser und/oder Ethanol sowie auch konzentrierte und wäßrige Essigsäure verwendbar.

Das Ausmaß der Umwandlung beträgt unter diesen Bedingungen max. 20 % (an (+)-Asocainol). Trotz Änderung der Versuchsparameter (Säuremenge, Konzentration, Lösungsmittel, Temperatur) konnte die Ausbeute an (+)-Asocainol nicht gesteigert werden, was wirtschaftlich keineswegs zufriedenstellen konnte.

Überraschenderweise wurde dann gefunden, daß bei dieser Umsetzung mit organischen Säuren durch Zugabe von Salzen und Oxidationsmitteln der Anteil an (+)-Asocainol im Reaktionsgemisch drastisch gesteigert werden kann. Im Gleichgewicht werden ca. 55-65 % (+)-Asocainol und 35-45 % R,S-Asocainol erhalten. Dieses Ergebnis entspricht dem aus Enthalpiewerten abgeschätzten Gleichgewicht und den maximal erreichten Anteilen beider Substanzen. Zur Erreichung des Gleichgewichts sind beides, Salz und Oxidationsmittel, unbedingt erforderlich. Jedes für sich allein hat keinen oder nur einen geringen Einfluß.

Die Reaktion kann auch in wässrigen Mineralsäuren durchgeführt werden.

Die gefundene Reaktion besitzt einen weiten Spielraum bezüglich der sie beeinflussenden Reaktionsparameter. Im folgengen wird dieser Spielraum näher beschrieben:

Lösungsmittel/ Säure: Geeignete Lösungsmittel sind wässrige Lösungen entweder anorganischer oder organischer Säuren, sowie deren Gemische, gegebenenfalls unter Zusatz polarer organischer Lösungsmittel (10-20 Vol%).
Beispielsweise sind wässrige Salzsäure, Schwefelsäure Phosphorsäure und Borsäure bevorzugte anorganische Säuren. Diese Säuren werden in Konzentrationen von 1 bis 3 Mol/l eingesetzt. Bei starken Säuren beträgt die Konzentration vorzugsweise 2 Mol/l und bei schwachen Säuren 5 Mol/l.
Neben Trifluoressigsäure (in Verdünnung mit Ethanol) ist als organische Säure Essigsäure vorzüglich als Lösungsmittel geeignet. Essigsäure ist sowohl wasserfrei als auch als wäßrige Lösung einzusetzen. Der Anteil Wasser kann zwischen 0 % und 90 % variiert werden. Bevorzugt wird 50-60 %ige Essigsäure eingesetzt. Es wird angenommen, daß auch andere polare Lösungsmittel, wie niedere Alkohole mit 1-10 C-Atomen, Ketone mit 1-10 C-Atomen, beispielsweise Aceton, Ether, wie Diethylether, Dioxan, Tetrahydrofuran, Dimethylformamid, Dimethylsulfoxid für die Umsetzung brauchbar sind. Anstelle von Trifluoressigsäure oder Essigsäure können auch andere starke bis mittelstarke organische Säuren eingesetzt werden. Ameisensäure, Hydroxyessigsäure, Propionsäure, Malonsäure, Benzoesäure, Oxalsäure Citronensäure, Weinsäure und Milchsäure seien beispielhaft genannt, ohne dadurch die Auswahl beschränken zu wollen. Die Säuren werden als 10-60%ige Lösung, vorzugsweise in 40-60 Gew% eingesetzt. Dem entsprechen etwa 1 - 20, vorzugsweise 5 - 15 Äquivalente Asocainol.

Konzentration: Die Konzentration von Asocainol in der Lösung (≦ 20 %) ist nicht entscheidend für das Ergebnis. Aus Gründen der praktischen Durchführbarkeit, - auch hinsichtlich des technischen Maßstabes -, bieten sich Konzentrationen zwi-

| | |
|---|---|
| | schen 10 und 20 % an. |
| Temperatur | Vernünftige Reaktionsgeschwindigkeiten werden erst ab 60 °C Reaktionstemperatur erzielt. Zwischen 60 °C und Rückflußtemperatur (118 °C in konzentrierter Essigsäure) ist jede Temperatur wählbar. Bevorzugt wird eine Temperatur um 80 °C als Kompromiß zwischen Reaktionsgeschwindigkeit und Vermeidung von Nebenreaktionen. |
| Salze | Für die Reaktion können vielerlei Salze verwendet werden, vorausgesetzt, sie sind im Lösungsmittel nicht völlig unlöslich. Getestet wurden Alkali-und Erdalkalimetallhalogenide, -sulfate und -acetate, Ammoniumhalogenide und -acetat, sowie Übergangsmetallsalze wie $FeCl_2$, $FeCl_3$, CuCl, $CuCl_2$, $CuSO_4$, Cu(II)-acetat, $ZnCl_2$, $NiCl_2$, Ni(II)acetylacetonat, V(III)acetylacetonat, Fe(III)-acetylacetonat.<br>Das preiswerte Natriumchlorid ergab neben den ebenfalls guten Salzen NaBr, KCl, KBr, LiCl und LiBr das beste Ergebnis.<br>Die verwendete Salzmenge liegt zwischen 10 Mol-% und 400 Mol-%, vorzugsweise bei ca. 30-80 Mol-% bezogen auf Asocainol. Bei Konzentrationen unter 10 Mol-% wird die Reaktionsgeschwindigkeit deutlich verlangsamt (3fache Reaktionsdauer), über 400 Mol-% hinausgehende Salzmengen werden nicht mehr vollständig gelöst oder führen teilweise durch Bildung zweier Phasen infolge des sog. "Salzeffektes" zu Problemen. |
| Oxidationsmittel | Es wird angenommen, daß das Oxidationsmittel durch einen reversiblen Redoxprozeß am $C_6$-Atom oder am benachbarten Stickstoffatom eine Aktivierung der $C_6$-Stellung bewirkt.<br>Katalytische Mengen (1-3 Mol%) des Oxidationsmittels sollten deshalb zur Gleichgewichtseinstellung ausreichen. Um unvermeidbare oxidative Nebenreaktionen zu kompensieren und eine ausreichende Reaktionsgeschwindigkeit zu ermöglichen, ist es jedoch vorteilhaft, größere Mengen einzusetzen (10-50 Mol%).<br>Die Reaktion läuft überraschend leicht mit mittelstarken Oxidationsmitteln, während mit schwachen Oxidationsmitteln die Reaktion zu langsam verläuft und die Gleichgewichtsverhältnisse nicht erreicht werden. Starke Oxidationsmittel führen dagegen durch Überoxidation zu hohen Substanzverlusten und sind somit als Katalysator ungeeignet. In Luft, Wasserstoffperoxid und Persäuren wurden ideale Oxidationsmittel gefunden, welche die beschriebenen Nachteile nicht aufweisen. Vor allem Luft und Wasserstoffperoxid sind die Oxidationsmittel der Wahl.<br>Die Oxidationsmittel Luft und Wasserstoffperoxid sind bereits in katalytischen Mengen ab 3 Mol% wirksam. Wegen der schnellen Zersetzung von Wasserstoffperoxid sind zur Vervollständigung der Reaktion ca. 20 - 30 Mol% erforderlich. Stoechiometrische Mengen beider Oxidationsmittel sind nachteilig, da hierbei Substanzverlust durch oxidative Nebenreaktionen eintritt.<br>Salze, welche gleichzeitig auch Oxidationsmittel darstellen, wie z. B. $CuCl_2$, Cu-(II)acetat, $FeCl_3$, usw. können gleichzeitig als Salz und Oxidationsmittel verwendet werden. Mit $CuCl_2$ (50 Mol%) wurden beispielsweise Ausbeuten von 40-50% (+)-Asocainol im Reaktionsgemisch erreicht. Ihre Benutzung ist jedoch aus ökologischen Gründen problematisch. Ihre zum Teil vorhandene Fähigkeit weitere anders geartete Raktionen eingehen zu können wie z. B. Chlorierung durch $CuCl_2$ oder $FeCl_3$ schränkt die Anwendung weiter ein.<br>Die untersuchten Oxidationsmittel können anhand ihrer Redoxpotentiale geordnet werden. Bis zu einem Redoxpotential von ca. 1 Volt ergeben die Oxidationsmittel keinen oder nur geringfügigen Substanzverlust durch Überoxidation. Es wird angenommen, daß auch vereinzelt Oxidationsmittel mit einem höheren Redoxpotential eingesetzt werden können, wie z. B. $NaJO_3$ und $MnO_2$. |

Unter diesen Reaktionsbedingungen läßt sich nicht nur das R,S-Isomere in das R,R-Isomere umwandeln, sondern auch die übrigen Isomeren sowie die Racemate. Die Beziehungen der ineinander umwandelbaren Isomeren und Racemate sind im folgenden dargestellt:

R,R $\rightleftarrows$ R,S

S,R $\rightleftarrows$ S,S

(R,R + S,S) ⇄ (R,S + S,R) Racemat

Zusammen mit der in der in der DE-P 34 19 099.7 beschriebenen thermischen Isomerisierung, durch die R,S in S,S und S,R in R,R umgewandelt werden kann, läßt sich nunmehr jedes gewünschte Isomere herstellen.

Dadurch, daß das vorher nur begrenzt durch die Umwandlung in (-)-Asocainol nutzbare Nebenprodukt, das R,S-Isomere nunmehr auch in (+)-Asocainol umgewandelt werden kann, wird das gesamte, durch "teures" Thebain eingebrachte Substanzmaterial zur Herstellung des Racemats (±)-Asocainol (R,R + S,S) nutzbar. Neben der dadurch erzielten Reduzierung der Herstellungskosten stellt dieses Verfahren auch eine einfache, schnelle und auch im Großmaßstab durchführbare Methode dar, um alle bei Totalsynthesen anfallenden Isomere zur Umwandlung in (±)-Asocainol zu nutzen.

Das beanspruchte Verfahren wird im folgenden anhand einiger Beispiele näher beschrieben.

Herstellung von (+)-6,7,8,9-Tetrahydro-2,12-dimethoxy-7-methyl-6(e')-phenethyl-5H-dibenz(d,f)azonin-1-ol Hydrochlorid (R,R-Asocainol-HCl)

**Beispiel 1**

In einem 10 l-Reaktionsgefäß werden 1 kg (2,4 Mol) R,S-Asocainol in 4,3 Liter 54 %iger Essigsäure aufgelöst. Nach Zusatz von 500 g (8,5 Mol) NaCl wird das Gemisch zum Rückfluß erhitzt und Luft (Preßluft) in leichtem Strom über oder in die Lösung geleitet. Nach 1,5 Stunden ist die Reaktion beendet. Im Vakuum wird anschließend alles zur Trockene eingeengt. Die Zusammensetzung des Rückstandes wird mittels HPLC untersucht. Der Gehalt an R,R-Asocainol beträgt 55-60 %, an R,S-Asocainol 40-45 %.

Der erhaltene Rückstand wird in 5 Liter Wasser und 5 Liter Methylenchlorid aufgenommen, mit konzentrierter Ammoniaklösung auf pH 8-9 eingestellt und die Phasen getrennt. Die wäßrige Phase wird nochmals mit insgesamt 4 Liter Methylenchlorid ausgerührt. Die vereinigten organischen Phasen werden mit Wasser neutralgewaschen und anschließend über einem Gemisch aus Natriumsulfat und 100 g Bleicherde getrocknet. Nach dem Filtrieren werden zum Filtrat 69,8 g HCl-Gas (= 80 % d. Th.) in 400 ml Isopropanol zugegeben. Nach Zugabe weiterer 4 Liter Isopropanol wird im leichten Vakuum bei 50° C alles Methylenchlorid entfernt, die Fällung von R,R-Asocainol-HCl durch Rühren während einer Stunde im Eisbad vervollständigt und das Kristallisat abfiltriert, mit Isopropanol nachgewaschen und im Umlufttrockenschrank bei 70-90° C getrocknet.

Man erhält 512 g (+)-6,7,8,9-Tetrahydro-2,12-dimethoxy-7-methyl-6(e')-phenethyl-5H-dibenz(d,f)azonin-1-ol Hydrochlorid (R,R-Asocainol-HCl) = 47,2 % d. Th.

Schmp.: 230° C; $[\alpha]_D$ = + 17,6° (c = 1/$H_2O$);

HPLC-Gehalt: 99 %

Aus der Mutterlauge der obigen Hydrochloridfällung wird durch Ammoniaklösung die Base freigesetzt, diese wird isoliert und aus Methylenchlorid-Isopropanol umgefällt. Man erhält so 413 g durch R,R-Asocainol verunreinigtes R,S-Asocainol zurück (41 % d. Th.)

Schmp.: 135° C; HPLC-Gehalt: 93,5 % R,S-Asocainol und 5,4 % R,R-Asocainol.

Die Mischung kann ohne weitere Reinigung erneut zur Umlagerung eingesetzt werden.

**Beispiel 2**

20 g (0,048 Mol) R,S-Asocainol werden in 100 ml 60 %iger Essigsäure gelöst, mit 2 g (0,034 Mol) NaCl versetzt und auf 80° C aufgeheizt. Unter gutem Rühren wird während 1,5 Stunden Luft über die Lösung geleitet. Nach beendeter Reaktion wird im Vakuum alles zur Trockene eingeeingt. Der Rückstand setzt sich nach HPLC zusammen aus 53 % R,R-Asocainol und 47 % R,S-Asocainol. Man arbeitet analog Beispiel 1 auf und erhält so 9,5 g R,R-Asocainol-HCl (44 % d. Th.).

Schmp. 236,2° C; HPLC-Gehalt: 95 %

Durch Umfällen aus Methylenchlorid-Isopropanol wird das Produkt rein erhalten.

**Beispiel 3**

20 g (0,048 Mol) R,S-Asocainol werden unter Stickstoff in 100 ml 60 %iger Essigsäure mit 2 g (0,034 Mol) NaCl auf 80° C erwärmt. Während einer Stunde werden nach und nach 469 mg (0,0138 Mol) Wasserstoffperoxid in 2,5 ml Wasser zugegeben. Man läßt 0,5 Stunden bei 80° C nachreagieren und arbeitet analog Beispiel 1 auf. Der Rückstand setzt sich nach HPLC zusammen aus 52 % R,R-Asocainol und 48 % R,S-Asocainol.

**Beispiel 4**

10 g (0,024 Mol) R,S-Asocainol und 1 g (0,017 Mol) NaCl werden mit 2 ml 35 %iger Salzsäurelösung (0,023 Mol) versetzt, in 50 ml 80 %iger Essigsäure gelöst und auf 80 °C erwärmt. Nach Zugabe von 40,6 mg (0,0012 Mol) Wasserstoffperoxid in 0,36 ml Wasser wird 1 Stunde gerührt und nochmals 40 mg Wasserstoffperoxid in 0,36 ml Wasser zugegeben. Man läßt 2 Stunden bei 80 °C nachreagieren, arbeitet analog Beispiel 1 auf und erhält 4 g R,R-Asocainol-HCl (37 %)
$[\alpha]_D$ = + 17,6 °C (c = 1,02/$H_2O$).

Durch Umfällen aus Methylenchlorid-Isopropanol wird das Produkt rein erhalten.

Herstellung von (±)-6,7,8,9-Tetrahydro-2,12-dimethoxy-7-methyl-6(a')phenethyl-5H-dibenz(d,f)azonin-1-ol (R,S + S,R-Racemat)

**Beispiel 5**

10 g (0,024 Mol) R,S-Asocainol und 1 g (0,017 Mol) NaCl werden in 50 ml 3 molarer wässriger Phosphorsäure gelöst und auf 90 °C erwärmt. Nach Zugabe von 50 mg (0,0014 Mol) Wasserstoffperoxid (1 ml einer 5%igen Lösung) wird 1 Stunde gerührt und nochmals 50 mg Wasserstoffperoxid (1 ml einer 5%igen Lösung) zugegeben. Nach 1 Stunde Nachreaktion bei 90 °C wird analog Beispiel 1 aufgearbeitet.

Nach HPLC enthält der Rückstand 42 % R,R-Asocainol und 51,4 % R,S-Asocainol.

Durch Umfällen aus Methylenchlorid-Isopropanol wird R,R-Asocainol-HCl rein erhalten.

**Beispiel 6**

10 g (0,024 Mol) R,S-Asocainol und 1 g (0,017 Mol) NaCl werden in 50 ml 80%iger Essigsäure gelöst, mit 3,09 g (0,05 Mol) Borsäure versetzt und auf 80 °C erwärmt. Nach Zugabe von 50 mg (0,0014 Mol) Wasserstoffperoxid (1 ml einer 5%igen Lösung) wird 30 Minuten gerührt und nochmals 50 mg Wasserstoffperoxid (1 ml einer 5%igen Lösung) zugegeben. Man läßt 30 Minuten bei 80 °C nachreagieren und arbeitet analog Beispiel 1 auf.

Der Rückstand enthält nach HPLC 57 % R,R-Asocainol und 39 % R,S-Asocainol.

**Beispiel 7**

10,5 g (0,025 Mol) racemisches R,R + S,S-Asocainol werden in 50 ml 60 %iger Essigsäure gelöst, mit 1 g (0,017 Mol) NaCl versetzt und auf Rückfluß erhitzt. Während 2 Stunden wird Luft in die Lösung geleitet. Nach beendeter Reaktion wird der Ansatz im Vakuum eingeengt. Der Rückstand enthält nach HPLC 62 % Ausgangsprodukt und 38 % R,S + S,R-Racemat. Die Aufarbeitung erfolgt analog Beispiel 1 durch Fällung mit HCl. Man erhält 5,9 g Ausgangsprodukt als Hydrochlorid (52 %) zurück.
Schmp.: 213 °C; HPLC-Zusammensetzung: 94,5 % R,R + S,S-Racemat und 5,5 % R,S + S,R-Racemat.

Aus der Mutterlauge der obigen Fällung wird mit konzentrierter Ammoniaklösung die Base freigesetzt und mit Methylenchlorid extrahiert. Die organische Phase wird mit Wasser neutral gewaschen und über $Na_2SO_4$ und Bleicherde getrocknet. Die Lösung wird filtriert und anschließend das Methylenchlorid im leichten Vakuum entfernt. Der ausgefallene Rückstand setzt sich nach HPLC zusammen auf ca. 5 % R,R + S,S-Racemat und ca. 94 % R,S + S,R-Racemat.
Ausbeute ca. 4 g (38 %).

Zur Reinigung wird die Base ins Hydrochlorid überführt und aus salzsaurer wäßriger Lösung umkristallisiert.

## Patentansprüche

1. Verfahren zur Isomerisierung von [R,R; S,S; S,R; R,S; (R,R + S,S); (R,S + S,R)]-Asocainol-Isomeren in 6-Position, dadurch gekennzeichnet, daß die Isomeren bei einer Temperatur von 60 °C bis Rückflußtemperatur in einem eine anorganische oder organische Säure enthaltenden polaren Lösungsmittel mit mindestens katalytischen Mengen entweder eines Salzes und eines Oxidationsmittels oder eines oxidierend wirkenden Salzes behandelt werden.

2. Verfahren nach Anspruch 1, dadurch gekennzeichnet, daß anorganische oder organische Salze der

Alkali-, Erdalkali-oder Übergangsmetalle vor allem Lithium-, Natrium-, Kaliumchlorid sowie Lithium-, Natrium- oder Kaliumbromid eingesetzt werden.

3. Verfahren nach Anspruch 1 oder 2, dadurch gekennzeichnet, daß 0,1-4 Mol Salz, vorzugsweise 0,3-0,8 Mol Salz pro Mol Asocainol verwendet werden.

4. Verfahren nach einem der Ansprüche 1-4, dadurch gekennzeichnet, daß ein mittelstarkes Oxidationsmittel in einer Menge von 0,01-0,5 Mol, vorzugsweise 0,03-0,3 Mol pro Mol Asocainol verwendet wird.

5. Verfahren nach einem der Ansprüche 1-4, dadurch gekennzeichnet, daß als Oxidtionsmittel Luftsauerstoff, Wasserstoffperoxid oder Peressigsäure in 3-30 Mol-% verwendet werden.

6. Verfahren nach einem der Ansprüche 1, 3 oder 4, dadurch gekennzeichnet, daß als oxidierend wirkendes Salz ein Cu(II)- oder Fe(III)-Salz verwendet wird.

7. Verfahren nach einem der Ansprüche 1-6, dadurch gekennzeichnet, daß die Säure eine mittelstarke bis starke Säure ist und in einer Menge von 1-20 Äquivalenten, bezogen auf Asocainol, verwendet wird.

8. Verfahren nach einem der Ansprüche 1-7, dadurch gekennzeichnet, daß als Lösungsmittel wäßrige oder alkoholische Lösungen der Essigsäure oder Trifluoressigsäure verwendet werden.

9. Racemisches (R,S + S,R)-Asocainol.

**Claims**

1. Process for the isomerization of [R,R; S,S; S,R; R,S; (R,R + S,S); (R,S + S,R)]-asocainol isomers in the 6-position, characterized in that the isomers are treated at a temperature of 60 °C up to reflux temperature in a polar solvent containing an inorganic or organic acid with at least catalytic quantities either of a salt and an oxidation agent or of a salt with an oxidizing activity.

2. Process according to claim 1, characterized in that inorganic or organic salts of the alkali metals, alkaline earth metals or transition metals, above all lithium chloride, sodium chloride, potassium chloride as well as lithium bromide, sodium bromide or potassium bromide are used.

3. Process according to claim 1 or 2, characterized in that 0.1-4 mole salt, preferably 0.3-0.8 mole salt per mole asocainol are used.

4. Process according to one of claims 1-4, characterized in that a medium-strong oxidation agent in a quantity of 0.01-0.5 mole, preferably 0.03-0.3 mole per mole asocainol is used.

5. Process according to one of claims 1-4, characterized in that as oxidation agent there are used atmospheric oxygen, hydrogen peroxide or peracetic acid in 3-30 mole %.

6. Process according to one of claims 1, 3 or 4, characterized in that a Cu(II)-salt or Fe(III)-salt is used as salt with an oxidizing activity.

7. Process according to one of claims 1-6, characterized in that the acid is a medium-strong to strong acid and is used in a quantity of 1-20 equivalents relative to asocainol.

8. Process according to one of claims 1-7, characterized in that aqueous or alcoholic solutions of the acetic acid or trifluoroacetic acid are used as solvents.

9. Racemic (R,S + S,R)-asocainol.

**Revendications**

1. Procédé d'isomérisation des isomères {R,R; S,S; S,R; R,S; (R,R + S,S); (R,S + S,R)} de l'asocaïnol en position-6, caractérisé en ce que les isomères sont traités, à une température comprise entre 60 °C

et la température de reflux, dans un solvant polaire contenant un acide minéral ou organique, par des quantités au moins catalytiques d'un sel et d'un oxydant, ou d'un sel à effet oxydant.

2. Procédé selon la revendication 1, caractérisé en ce qu'on utilise des sels organiques ou minéraux des métaux alcalins, des métaux alcalino-terreux ou des métaux de transition, surtout le chlorure de lithium, de sodium ou de potassium ou le bromure de lithium, de sodium ou de potassium.

3. Procédé selon la revendication 1 ou 2, caractérisé en ce qu'on utilise 0,1-4 moles de sel, de préférence 0,3-0,8 mole de sel par mole d'asocaïnol.

4. Procédé selon l'une des revendications 1 à 4, caractérisé en ce qu'on utilise un oxydant moyennement fort en une quantité de 0,01 à 0,5 mole, de préférence de 0,03 à 0,3 mole par mole d'asocaïnol.

5. Procédé selon l'une des revendications 1 à 4, caractérisé en ce qu'on utilise comme oxydant, l'oxygène de l'air, du peroxyde d'hydrogène ou de l'acide peracétique, en une quantité de 3 à 30% en moles.

6. Procédé selon l'une des revendications 1, 3 ou 4, caractérisé en ce qu'on utilise comme sel à effet oxydant, un sel de Cu(II) ou de Fe(III).

7. Procédé selon l'une des revendications 1 à 6, caractérisé en ce que l'acide est un acide moyennement fort à fort et est utilisé en une quantité de 1 à 20 équivalents par rapport à l'asocaïnol.

8. Procédé selon l'une des revendications 1 à 7, caractérisé en ce qu'on utilise comme solvant des solutions aqueuses ou alcooliques de l'acide acétique ou de l'acide trifluoroacétique.

9. Asocaïnol-(R,S + S,R) racémique.